# EUROPÄISCHE PATENTSCHRIFT

(11) **EP 4 663 743 B1**
(45) Veröffentlichungstag und Bekanntmachung des Hinweises auf die Patenterteilung: **29.07.2026**
(21) Anmeldenummer: 24182342.6
(22) Anmeldetag: 14.06.2024
(51) Int. Cl.: C12M 3/00, C12M 3/06, C12M 1/00

(54) **MODULARE TRÄGEREINHEIT UND MODULEINHEIT FÜR EINEN INKUBATOR SOWIE VERWENDUNG DIESER IN EINEM INKUBATOR UND INKUBATOR**
MODULAR TRAY UNIT AND MODULAR UNIT FOR INCUBATOR, AND INCUBATOR USING IT, AND INCUBATOR
UNITÉ DE SUPPORT MODULAIRE ET UNITÉ MODULAIRE POUR INCUBATEUR ET INCUBATEUR UTILISANT LADITE UNITÉ

(43) Veröffentlichungstag der Anmeldung: 17.12.2025
(73) Patentinhaber: Fraunhofer-Gesellschaft zur Förderung der angewandten Forschung e.V., 80686 München (DE); Life & Brain GmbH, 53127 Bonn (DE)
(72) Erfinder: Nießing, Bastian, 52511 Geilenkirchen (DE); Werner, Mario, 29345 Unterlüß (DE); Breitkreuz, Yannik, 53113 Bonn (DE)
(74) Vertreter: Eisenführ Speiser

(56) Entgegenhaltungen:
- EP-A1- 4 253 523
- WO-A1-2017/079682
- WO-A1-2024/038280
- CN-A- 106 536 711
- CN-A- 113 322 183
- CN-U- 220 413 391
- DE-B3- 102016 208 552
- DE-B4- 102010 060 635
- US-A1- 2020 010 792
- US-A1- 2024 141 280
- US-B1- 10 376 889
- US-B2- 9 226 494

## Beschreibung

Die Erfindung betrifft eine modulare Trägereinheit für einen Inkubator. Ferner betrifft die Erfindung eine Moduleinheit für einen Inkubator sowie einen Inkubator.

Ein Inkubator ist ein Zellkultivierungssystem. Zur Kultivierung von Zellen ist ein Inkubator ein Wärme- oder Brutschrank, auch bekannt als Brutapparat oder medizinisches Temperiergerät. In den Lebenswissenschaften wird solch ein Inkubator für das Kultivieren von Zell- und Gewebekulturen verwendet. Mit dem Inkubator lässt sich ein definiertes bzw. gewünschtes Mikroklima einstellen, um biologische Prozesse und Reaktionen in-vitro zu untersuchen. Entsprechende In-vitro-Untersuchungen werden beispielsweise in der Biologie, Medizin, Pharmakologie oder Lebensmittel- und Kosmetikindustrie genutzt. Diese In-vitro-Untersuchungen finden außerhalb eines lebenden Organismus in einer kontrollierten künstlichen Umgebung statt. In der Biologie und Medizin werden beispielsweise aus Stammzellen abgeleitete Zellsysteme in den Bereichen Krankheitsmodellierung, Arzneimittelentwicklung sowie patientenspezifische Medizin genutzt.

In ersten In-vitro-Studien lag der Schwerpunkt auf der Verwendung spezifischer Zelltypen in einem zweidimensionalen Verbund. In der Zwischenzeit werden immer häufiger Zellen im dreidimensionalen Aggregat (auch als Organoide/Sphäroide bezeichnet) eingesetzt, da sie komplexere Strukturen mit verschiedenen Zelltypen ausbilden und somit die In-vivo-Situation (innerhalb eines lebenden Organismus) näher abbilden. Zur Bildung und Kultivierung solcher 3D-Zellaggregate werden verschiedene Methoden genutzt. Meist werden nach der Formierung der Zellaggregate sich bewegende/rotierende Inkubatoren, wie zum Beispiel Bioreaktoren, verwendet, um die 3D-Zellaggregate in Bewegung/Levitation zu halten. Dies soll ein Zusammenschließen der einzelnen 3D-Zellaggregate verhindern und die Nährstoffversorgung verbessern.

Viele bekannte Bioreaktoren sind sowohl in der Anschaffung als auch während der Nutzung teuer, eignen sich jedoch nicht für jede wissenschaftliche Fragestellung. So umfassen beispielsweise viele Bioreaktoren Kulturgefäße mit einem Mindestvolumen von 100 ml und mehr. Dies ist für die Skalierung des Herstellungsprozesses von 3D-Zellaggregaten hilfreich, in der Etablierungsphase werden nach Erkenntnis der Erfinder jedoch in aller Regel Kulturgefäße mit kleineren Volumina bevorzugt.

Darüber hinaus ist nach der Erkenntnis der Erfinder die maximale Anzahl von Kulturgefäßen zur parallelen bzw. zeitgleichen Zellkultivierung von 3D-Zellaggregaten in Bioreaktoren beschränkt. So können mit kommerziell verfügbaren Bioreaktoren zur Kultivierung von 3D-Zellaggregaten allenfalls vier Kulturgefäße parallel genutzt werden. Sollen größere Patienten-Kohorten oder eine Vielzahl verschiedener Arzneimittelkonzentrationen parallel untersucht werden, reicht nach der Erkenntnis der Erfinder die Kapazität der am Markt verfügbaren Inkubatoren nicht aus. Wenn mehrere Untersuchungen parallel durchgeführt werden sollen, müssen zusätzliche Bioreaktoren angeschafft werden. Dies ist kosten- und platzintensiv.

Als ein beispielhafter Bioreaktor zur Kultivierung von 3D-Zellaggregaten ist den Erfindern ein in sich geschlossenes Tischgerät mit manueller Temperatur- und CO₂-Regelung bekannt. Nach der Erkenntnis der Erfinder können bei diesem Tischgerät Zellen in bis zu vier Kulturgefäßen mit einem Kultivierungsvolumen von bis zu 50 ml parallel rotierend über separat ansteuerbare Linearmotoren kultiviert werden. Die hierzu eingesetzten Kulturgefäße weisen einen flachen Boden und im unteren Drittel vertikale Strömungsbrecher (Rippen) unterschiedlicher Größe auf, die bei reduzierten Scherkräften eine sanfte Verwirbelung bewirken. Die Rotationsparameter und weitere Zellkulturbedingungen können vom Benutzer über eine Bedieneinheit des Bioreaktors für jedes Kulturgefäß individuell eingestellt werden. Die Temperatur und der CO₂-Gehalt werden für den gesamten Bioreaktor eingestellt und sind nicht individuell für jedes Kulturgefäß einstellbar. Mehr als vier Kulturen gleichzeitig können mit diesem Tischgerät nicht kultiviert werden. Hinzu kommt, dass bei diesem Tischgerät die Einstellung des Gehalts weiterer Gase, wie etwa Sauerstoff, und die Regelung der Luftfeuchtigkeit nicht möglich sind.

Prinzipiell sind auch Geräte bekannt, in denen mehr als vier Kulturgefäße eingesetzt werden, beispielsweise in 24er- oder 48er-Gebinden. Die Kulturgefäße haben dann aber nur ein Fassungsvolumen von meist 5 ml bis 15 ml und sind nicht für die Kultivierung von 3D-Zellaggregaten geeignet. Je größer das Fassungsvolumen der Kulturgefäße ist, desto geringer ist die Anzahl der Kulturgefäße, welche in einem Gerät gleichzeitig eingesetzt werden. Beispielsweise werden Kulturgefäße, die ein Fassungsvermögen von 250 ml oder 500 ml aufweisen, in Geräten verwendet, die auf single-use oder auf zwei oder vier Gefäße ausgelegt sind.

Insofern werden bei der Verwendung von Kulturgefäßen mit einem Fassungsvolumen von >15 ml in der Regel mehrere Geräte verwendet, die bis zu vier Kulturgefäße aufnehmen können, wenn der Kapazitätsbedarf vier Kulturgefäße überschreitet.

Unabhängig von der in einem Gerät eingesetzten Anzahl der Kulturgefäße ist es aber erforderlich, einerseits die zu kultivierenden Zellen in Bewegung zu halten und andererseits die Zellkulturbedingungen einzustellen.

WO 2024/038280 offenbart ein modulares Bioprozessier-System, wobei eine Mehrzahl an Bioprozess-Modulen (102) und ein Roboter (104) in einem Array in gestapelter Weise (in Reihen und Zeilen) in einem Inkubator (108) angeordnet sind. Dabei können die unterschiedlichen Module unterschiedliche Größe aufweisen und zu größeren Einheiten als 1x2, 2x2, 3x1 oder 4x4 Moduleinheiten verbunden werden.

Insofern liegt der Erfindung die Aufgabe zugrunde, eine Lösung bereitzustellen, welche die zuvor genannten Nachteile überwindet. Insbesondere liegt der Erfindung die Aufgabe zugrunde, eine modulare Trägereinheit und eine Moduleinheit für einen Inkubator, deren Verwendung in einem Inkubator sowie einen Inkubator bereitzustellen, welche die zuvor genannten Nachteile überwinden. Vorzugsweise liegt der Erfindung die Aufgabe zugrunde, eine modulare Trägereinheit und eine Moduleinheit für einen Inkubator, deren Verwendung in einem Inkubator sowie einen Inkubator bereitzustellen, welche eine kostengünstige und platzsparende sowie gleichzeitig am Kapazitätsbedarf der Kulturgefäße orientierte Zellkultivierung ermöglichen.

Gemäß einem ersten Aspekt wird die eingangs genannte Aufgabe durch eine modulare Trägereinheit nach Anspruch 1 gelöst. Die modulare Trägereinheit ist eine modulare Trägereinheit für einen Inkubator. Vorzugsweise ist die modulare Trägereinheit eine modulare Trägereinheit für eine Moduleinheit eines Inkubators.

Die modulare Trägereinheit gemäß dem ersten Aspekt umfasst einen Trägerkörper und eine Antriebsübertragungseinheit, die zur Übertragung eines Antriebsdrehmoments ausgebildet ist und an dem Trägerkörper angeordnet ist.

Der Trägerkörper weist eine Aufnahmeöffnung auf, die zur Aufnahme eines Kulturgefäßes oder einer Antriebseinheit ausgebildet ist, und weist drei, vier oder mehrere Seitenflächen auf, wobei mindestens eine der Seitenflächen eine erste Verbindungsschnittstelle aufweist, die zur Verbindung der modularen Trägereinheit mit einer weiteren modularen Trägereinheit ausgebildet ist.

Der Trägerkörper ist vorzugsweise einteilig ausgebildet.

Alternativ kann es bevorzugt sein, den Trägerkörper mehrteilig auszubilden. In dieser mehrteiligen Ausführungsform kann der Trägerkörper mehrere Trägerkörperelemente umfassen. Insbesondere kann es bevorzugt sein, dass der Trägerkörper je Seitenfläche ein Trägerkörperelement aufweist. In dieser mehrteiligen Ausführungsform wird die Aufnahmeöffnung durch die Trägerkörperelemente ausgebildet. Ergänzend oder alternativ kann es ferner bevorzugt sein, dass ein oder mehrere Trägerkörperelemente die drei, vier oder mehr Seitenflächen ausbilden oder umfassen und ein oder mehrere Trägerkörperelemente die Aufnahmeöffnung ausbilden oder umfassen.

Es kann bevorzugt sein, dass der Trägerkörper und/oder die Trägerkörperelemente die Seitenflächen integral ausbilden. Ergänzend oder alternativ kann es bevorzugt sein, dass der Trägerkörper und/oder die Trägerkörperelemente die Aufnahmeöffnung integral ausbilden.

Es kann bevorzugt sein, dass ein Abschnitt des Trägerkörpers, der die drei, vier oder mehreren Seitenflächen aufweist, eine Form und/oder einen Querschnitt aufweist, der von einer Form und/oder einem Querschnitt eines anderen Abschnitts des Trägerkörpers verschieden ist. Es kann beispielsweise bevorzugt sein, dass der Trägerkörper abschnittsweise zylinderförmig und/oder kegelförmig ausgebildet ist und ein anderer Abschnitt des Trägerkörpers die drei, vier oder mehr Seitenflächen umfasst oder ausbildet.

Es ist bevorzugt, dass der Trägerkörper eine erste Stirnfläche ausbildet. In einem Einbau- oder Betriebszustand der modularen Trägereinheit bildet diese erste Stirnfläche eine Unterseite bzw. den Boden des Trägerkörpers. Es kann bevorzugt sein, dass die erste Stirnfläche die Aufnahmeöffnung ausbildet oder diese umfasst. Ergänzend oder alternativ ist es bevorzugt, dass der Trägerkörper eine zweite Stirnfläche ausbildet. In einem Einbau- oder Betriebszustand der modularen Trägereinheit bildet diese zweite Stirnfläche eine Oberseite des Trägerkörpers. Es kann bevorzugt sein, dass die zweite Stirnfläche die Aufnahmeöffnung ausbildet oder diese umfasst.

Vorzugsweise erstreckt sich die Aufnahmeöffnung ausgehend von der ersten Stirnfläche in den Trägerkörper hinein. Ergänzend oder alternativ erstreckt sich die Aufnahmeöffnung in bevorzugter Weise ausgehend von der zweiten Stirnfläche in den Trägerkörper hinein. Vorzugsweise erstreckt sich die Aufnahmeöffnung zwischen der ersten und zweiten Stirnfläche.

Vorzugsweise ist die Aufnahmeöffnung eine Ausnehmung innerhalb des Trägerkörpers. Vorzugsweise weist die Aufnahmeöffnung einen zylinderförmigen Querschnitt auf. Ergänzend oder alternativ weist die Aufnahmeöffnung einen polygonalförmigen Querschnitt auf. Ergänzend oder alternativ weist die Aufnahmeöffnung einen kegelförmigen Querschnitt auf. Ergänzend oder alternativ weist die Aufnahmeöffnung abschnittsweise einen zylinderförmigen Querschnitt und/oder abschnittsweise einen polygonalförmigen Querschnitt und/oder abschnittsweise einen kegelförmigen Querschnitt auf.

Die Aufnahmeöffnung ist vorzugsweise als Sackloch oder als Durchgangsloch ausgebildet.

In einem Einbau- oder Betriebszustand der modularen Trägereinheit bilden die Seitenflächen des Trägerkörpers in bevorzugter Weise die Seitenwände des Trägerkörpers. Entsprechend können in einer mehrteiligen Ausführung des Trägerkörpers, die Trägerkörperelemente mit den jeweiligen Seitenflächen die Seitenwände des Trägerkörpers ausbilden. Der Trägerkörper erstreckt sich vorzugsweise zumindest teilweise seitlich zwischen den Seitenflächen. Vorzugsweise umschließen die Seitenflächen zumindest einen Abschnitt des Trägerkörpers.

Vorzugsweise besteht der Trägerkörper aus Aluminium, Stahl, insbesondere Edelstahl, und/oder Kunststoff. Insbesondere umfasst der Trägerkörper Aluminium, Stahl, vorzugsweise Edelstahl, und/oder Kunststoff.

Vorzugsweise ist der Trägerkörper ein Gussteil, insbesondere ein Spritzgussteil. Insbesondere ist der Trägerkörper im Gießverfahren, vorzugsweise im Spritzgießverfahren, hergestellt. Es kann auch bevorzugt sein, den Trägerkörper als Schmiedeteil auszubilden.

Ergänzend oder alternativ kann es bevorzugt sein, dass insbesondere ausschließlich die Funktionsflächen zerspanend bearbeitet werden. Die Funktionsflächen sind insbesondere die drei, vier oder mehr Seitenflächen und/oder die Aufnahmeöffnung des Trägerkörpers. Insbesondere kann es bevorzugt sein, die drei, vier oder mehr Seitenflächen und/oder die Aufnahmeöffnung des Trägerkörpers durch ein zerspanendes Verfahren herzustellen bzw. es kann bevorzugt sein, dass die drei, vier oder mehr Seitenflächen und/oder die Aufnahmeöffnung des Trägerkörpers zerspanend hergestellt sind. Für die zerspanende Bearbeitung kommt beispielsweise das Drehen und/oder Fräsen und/oder Bohren in Betracht.

Die Antriebsübertragungseinheit weist mindestens eine erste Antriebsübertragungsschnittstelle auf, welche mit dem Kulturgefäß oder der Antriebseinheit koppelbar ist, und eine zweite Antriebsübertragungsschnittstelle, die mit einer zweiten Antriebsübertragungsschnittstelle einer Antriebsübertragungseinheit der weiteren modularen Trägereinheit oder der Antriebseinheit koppelbar ist.

Vorzugsweise ist die erste Antriebsübertragungsschnittstelle für eine kraftschlüssige und/oder formschlüssige Verbindung mit dem Kulturgefäß oder der Antriebseinheit ausgebildet. Vorzugsweise weist die erste Antriebsübertragungsschnittstelle Vorsprünge und/oder Ausnehmungen auf. Es kann vorgesehen sein, dass die Vorsprünge und/oder Ausnehmungen der ersten Antriebsübertragungsschnittstelle zur kraftschlüssigen und/oder formschlüssigen Kopplung mit dem Kulturgefäß oder der Antriebseinheit ausgebildet sind. Vorzugsweise kann die erste Antriebsübertragungsschnittstelle für eine Kopplung der modularen Trägereinheit mit dem Kulturgefäß oder der Antriebseinheit einen Gummiring oder ähnliches aufweisen. Insbesondere kann die erste Antriebsübertragungsschnittstelle zur Kopplung mit dem Kulturgefäß oder der Antriebseinheit als Schnapp- und/oder Klemm- und/oder Steckverbindung ausgebildet sein.

Vorzugsweise weist die erste Antriebsübertragungsschnittstelle mehrere Verbindungsabschnitte auf. Es kann bevorzugt sein, dass einer der Verbindungsabschnitte für eine formschlüssige Verbindung mit dem Kulturgefäß oder der Antriebseinheit ausgebildet ist und ein anderer der Verbindungsabschnitte für eine kraftschlüssige Verbindung mit dem Kulturgefäß oder der Antriebseinheit. Ergänzend oder alternativ kann es bevorzugt sein, dass zumindest einer oder jeder der Verbindungsabschnitte für eine kraft- und/oder formschlüssige Verbindung mit dem Kulturgefäß oder der Antriebseinheit ausgebildet ist.

Vorzugsweise ist die zweite Antriebsübertragungsschnittstelle für eine kraftschlüssige und/oder formschlüssige Verbindung mit der zweiten Antriebsübertragungsschnittstelle der Antriebsübertragungseinheit der weiteren modularen Trägereinheit oder der Antriebseinheit ausgebildet. Vorzugsweise weist die zweite Antriebsübertragungsschnittstelle Vorsprünge und/oder Ausnehmungen auf. Es kann vorgesehen sein, dass die Vorsprünge und/oder Ausnehmungen der zweiten Antriebsübertragungsschnittstelle zur kraftschlüssigen und/oder formschlüssigen Kopplung mit der zweiten Antriebsübertragungsschnittstelle der Antriebsübertragungseinheit der weiteren modularen Trägereinheit oder der Antriebseinheit ausgebildet sind. Vorzugsweise kann die zweite Antriebsübertragungsschnittstelle für eine Kopplung der modularen Trägereinheit mit der zweiten Antriebsübertragungsschnittstelle der Antriebsübertragungseinheit der weiteren modularen Trägereinheit oder der Antriebseinheit einen Gummiring oder ähnliches aufweisen. Insbesondere kann die zweite Antriebsübertragungsschnittstelle zur Übertragung des Antriebsdrehmoments als Zahnradgetriebe und/oder Riementrieb und/oder Kettentrieb und/oder elektromagnetischer Trieb ausgebildet sein.

Vorzugsweise weist die zweite Antriebsübertragungsschnittstelle mehrere Verbindungsabschnitte auf. Es kann bevorzugt sein, dass einer der Verbindungsabschnitte für eine formschlüssige Verbindung mit der zweiten Antriebsübertragungsschnittstelle der Antriebsübertragungseinheit der weiteren modularen Trägereinheit oder der Antriebseinheit ausgebildet ist und ein anderer der Verbindungsabschnitte für eine kraftschlüssige Verbindung mit der zweiten Antriebsübertragungsschnittstelle der Antriebsübertragungseinheit der weiteren modularen Trägereinheit oder der Antriebseinheit ausgebildet ist. Ergänzend oder alternativ kann es bevorzugt sein, dass zumindest einer oder jeder der Verbindungsabschnitte für eine kraft- und/oder formschlüssige Verbindung mit der zweiten Antriebsübertragungsschnittstelle der Antriebsübertragungseinheit der weiteren modularen Trägereinheit oder der Antriebseinheit ausgebildet ist.

Die Erfindung beruht auf der Erkenntnis der Erfinder, dass die Modularität eine am Kapazitätsbedarf der Kultivierungsgefäße orientierte kostengünstige und platzsparende Zellkultivierung ermöglicht.

Gemäß einer bevorzugten Ausführungsform weist die modulare Trägereinheit die Antriebseinheit auf, die an und/oder in der Aufnahmeöffnung an dem Trägerkörper befestigt ist und zur Übertragung der Drehbewegung von der Antriebseinheit auf die Antriebsübertragungseinheit mit der ersten Antriebsübertragungsschnittstelle gekoppelt ist. Die Antriebseinheit ist vorzugsweise ein Elektromotor, insbesondere ein Schrittmotor. Die Antriebseinheit ist vorzugsweise entsprechend der ersten Antriebsübertragungsschnittstelle und/oder der zweiten Antriebsübertragungsschnittstelle der Antriebsübertragungseinheit ausgebildet.

Ferner weist eine bevorzugte Fortbildung der modularen Trägereinheit eine drehbar in der Aufnahmeöffnung angeordnete Kulturgefäßaufnahmeeinheit auf, die zur Aufnahme des Kulturgefäßes ausgebildet ist und zur Übertragung der Drehbewegung von der Antriebsübertragungseinheit auf die Kulturgefäßaufnahmeeinheit mit der ersten Antriebsübertragungsschnittstelle gekoppelt ist.

In der Kulturgefäßaufnahmeeinheit sind vorzugsweise ein Kulturgefäß oder mehrere Kulturgefäße anordenbar oder angeordnet.

Darüber hinaus ist gemäß einer bevorzugten Fortbildung der modularen Trägereinheit die Kulturgefäßaufnahmeeinheit zur Aufnahme des Kulturgefäßes topfförmig und/oder kegelförmig ausgebildet. Ergänzend oder alternativ weist die Kulturgefäßaufnahmeeinheit zur Aufnahme des Kulturgefäßes ein Sackloch auf, das sich zwischen einer Öffnung und einem Boden erstreckt.

Nach einer bevorzugten Ausführungsform weist das Sackloch einen ersten Befestigungsabschnitt auf, der zur kraft- und formschlüssigen Verbindung mit dem Kulturgefäß ausgebildet ist. Ergänzend oder alternativ weist das Sackloch einen zweiten Befestigungsabschnitt auf, der zur kraft- und formschlüssigen Verbindung mit dem Kulturgefäß ausgebildet ist.

Weiterhin ist in einer bevorzugten Fortbildung der modularen Trägereinheit vorgesehen, dass der erste Befestigungsabschnitt am Boden und/oder im Bereich des Bodens des Sacklochs ausgebildet ist und/oder für eine formschlüssige Verbindung Vorsprünge und/oder Vertiefungen aufweist, und/oder dass der zweite Befestigungsabschnitt am und/oder im Bereich der Öffnung ausgebildet ist und/oder für eine kraftschlüssige Verbindung ein elastisches Befestigungselement, insbesondere einen Gummiring, aufweist wobei das Befestigungselement vorzugsweise in einer Nut angeordnet ist, die in einer Seitenwand des Sacklochs ausgebildet wird.

Gemäß einer bevorzugten Ausführungsform der modularen Trägereinheit weist mindestens eine der Seitenflächen eine zweite Verbindungsschnittstelle auf, die zur Verbindung der modularen Trägereinheit mit einer weiteren modularen Trägereinheit ausgebildet ist.

Ferner ist nach einer bevorzugten Ausführungsform der modularen Trägereinheit vorgesehen, dass die erste Verbindungsschnittstelle und/oder die zweite Verbindungsschnittstelle für eine formschlüssige und/oder kraftschlüssige Verbindung ausgebildet ist.

Hinsichtlich einer weiter bevorzugten Fortbildung der modularen Trägereinheit ist die erste Verbindungsschnittstelle von der zweiten Verbindungsschnittstelle verschieden ausgebildet. Alternativ ist es bevorzugt, dass die erste Verbindungsschnittstelle der zweiten Verbindungsschnittstelle entspricht.

Ferner ist gemäß einer bevorzugten Ausführungsform der modularen Trägereinheit die erste Verbindungsschnittstelle zur Verbindung mit einer zweiten Verbindungsschnittstelle der weiteren Trägereinheit ausgebildet. Ergänzend oder alternativ ist vorzugsweise vorgesehen, dass die zweite Verbindungsschnittstelle zur Verbindung mit einer ersten Verbindungsschnittstelle der weiteren Trägereinheit ausgebildet ist.

Gemäß einer weiteren bevorzugten Fortbildung der modularen Trägereinheit weist die erste Verbindungsschnittstelle zur Verbindung mit der weiteren Trägereinheit für eine formschlüssige Verbindung Vorsprünge und/oder Vertiefungen auf. Ergänzend oder alternativ weist die erste Verbindungsschnittstelle zur Verbindung mit der weiteren Trägereinheit für eine kraftschlüssige Verbindung einen Magnet und/oder ein Federsystem auf.

Ergänzend oder alternativ ist es gemäß dieser bevorzugten Fortbildung vorgesehen, dass die zweite Verbindungsschnittstelle zur Verbindung mit der weiteren Trägereinheit für eine formschlüssige Verbindung Vorsprünge und/oder Vertiefungen aufweist. Ergänzend oder alternativ weist die zweite Verbindungsschnittstelle zur Verbindung mit der weiteren Trägereinheit für eine kraftschlüssige Verbindung einen Magnet und/oder ein Federsystem auf.

Darüber hinaus weist eine bevorzugte Ausführungsform der modularen Trägereinheit einen Demontagegriff auf, der mit dem Trägerkörper zur Demontage einer mit einer weiteren modularen Trägereinheit verbundenen modularen Trägereinheit koppelbar ist, wobei der Trägerkörper vorzugsweise einen Griffbefestigungsabschnitt aufweist, der für eine form- und/oder kraftschlüssige Verbindung mit dem Demontagegriff ausgebildet ist.

Weiterhin ist der Trägerkörper gemäß einer bevorzugten Fortbildung der modularen Trägereinheit würfelförmig und/oder quaderförmig ausgebildet.

Nach einer weiteren bevorzugten Fortbildung der modularen Trägereinheit ist die Antriebsübertragungseinheit gegenüber dem Trägerkörper mittels einer Lagereinheit drehbar gelagert.

Gemäß einer bevorzugten Ausführungsform der modularen Trägereinheit ist die Antriebsübertragungseinheit als Zahnradgetriebe mit mindestens einem Zahnrad ausgebildet oder umfasst ein Zahnradgetriebe mit mindestens einem Zahnrad.

Ferner ist gemäß einer bevorzugten Ausführungsform der modularen Trägereinheit vorgesehen, dass die Antriebsübertragungseinheit als Riemen- und/oder Kettentrieb ausgebildet ist oder einen Riemen- und/oder Kettentrieb umfasst.

Gemäß einer bevorzugten Fortbildung der modularen Trägereinheit ist die Antriebsübertragungseinheit als elektromagnetischer Antrieb ausgebildet oder umfasst einen elektromagnetischen Antrieb.

Ferner ist gemäß einer bevorzugten Fortbildung der modularen Trägereinheit vorgesehen, dass das Zahnradgetriebe genau ein auf einer Welle angeordnetes Zahnrad aufweist, wobei die Welle gegenüber dem Trägerkörper drehbar gelagert ist, und die Welle an einem Ende die erste Antriebsübertragungsschnittstelle ausbildet, und das Zahnrad die zweiten Antriebsübertragungsschnittstellen ausbilden.

Nach einer bevorzugten Ausführungsform der modularen Trägereinheit weist das Zahnradgetriebe ein auf einer Zentralradwelle angeordnetes Zentral-Zahnrad auf und die Zentralradwelle bildet an einem Ende die erste Antriebsübertragungsschnittstelle aus, wobei die Zentralradwelle gegenüber dem Trägerkörper drehbar gelagert ist. Ergänzend ist vorgesehen, dass das Zahnradgetriebe je Seitenfläche mindestens ein Außen-Zahnrad aufweist, das in das Zentral-Zahnrad greift, wobei das mindestens eine Außen-Zahnrad mittels eines Außenradzapfens gegenüber dem Trägerkörper drehbar gelagert ist.

In einem zweiten Aspekt der Erfindung wird die eingangs genannte Aufgabe gelöst durch eine Moduleinheit nach Anspruch 14. Diese Moduleinheit weist mindestens zwei modulare Trägereinheit auf, wie diese hinsichtlich des ersten Aspekts zur modularen Trägereinheit bzw. hinsichtlich möglicher bevorzugter Ausführungsformen oder Fortbildungen der modularen Trägereinheit gemäß dem ersten Aspekt beschrieben worden sind.

Gemäß einer ersten bevorzugten Fortbildung der Moduleinheit weist mindestens eine der mindestens zwei modularen Trägereinheiten eine Antriebseinheit auf, wie diese gemäß einer bevorzugten Ausführungsform oben beschrieben wird, und weist mindestens eine der mindestens zwei modularen Trägereinheiten eine Kulturgefäßaufnahmeeinheit, insbesondere eine Kulturgefäßaufnahmeeinheit mit einem Kulturgefäß, auf, wie diese gemäß einer bevorzugten Ausführungsform oben beschrieben wird.

In einem dritten Aspekt der Erfindung wird die eingangs genannte Aufgabe gelöst durch einen Inkubator nach Anspruch 17. Dieser Inkubator weist mindestens eine modulare Trägereinheit gemäß dem ersten Aspekt bzw. möglichen bevorzugten Ausführungsformen oder Fortbildungen der modularen Trägereinheit gemäß dem ersten Aspekt auf. Ergänzend oder alternativ weist dieser Inkubator eine Moduleinheit gemäß dem zweiten Aspekt bzw. möglichen bevorzugten Ausführungsformen oder Fortbildungen der Moduleinheit gemäß dem zweiten Aspekt auf.

Gemäß einer bevorzugten Fortbildung weist der Inkubator eine Steuerungseinheit zur Steuerung der modularen Trägereinheit und/oder der Moduleinheit auf, wobei die Steuerungseinheit signaltechnisch und/oder steuerungstechnisch, insbesondere mittels einer Leitung kabelgebunden oder drahtlos, mit der modularen Trägereinheit und/oder der Moduleinheit gekoppelt ist.

In einem vierten Aspekt der Erfindung wird die eingangs genannte Aufgabe gelöst durch eine Verwendung der modularen Trägereinheit gemäß dem ersten Aspekt bzw. möglichen bevorzugten Ausführungsformen oder Fortbildungen der modularen Trägereinheit gemäß dem ersten Aspekt und/oder der Moduleinheit gemäß dem zweiten Aspekt bzw. möglichen bevorzugten Ausführungsformen oder Fortbildungen der Moduleinheit gemäß dem zweiten Aspekt in einem Inkubator.

Zu den Vorteilen, Ausführungsvarianten und Ausführungsdetails dieser weiteren Aspekte der Erfindung und ihrer Fortbildungen wird auch auf die vorangegangene Beschreibung zu den entsprechenden Merkmalen der modularen Trägereinheit sowie der jeweiligen anderen Aspekte verwiesen.

Ausführungsformen der Erfindung werden nun nachfolgend anhand der Zeichnungen beschrieben. Diese sollen die Ausführungsformen nicht notwendigerweise maßstäblich darstellen, vielmehr sind die Zeichnungen, wenn dies zur Erläuterung dienlich ist, in schematisierter und/oder leicht verzerrter Form ausgeführt. Im Hinblick auf Ergänzungen der aus den Zeichnungen unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in den Zeichnungen sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, den Zeichnungen und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsformen oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsformen sowie anhand der Zeichnungen; diese zeigen in:
- Fig. 1: eine schematische Darstellung eines Inkubators gemäß einer bevorzugten Ausführungsform;
- Fig. 2: eine schematische Darstellung einer Seitenansicht einer modularen Trägereinheit gemäß einer bevorzugten Ausführungsform;
- Fig. 3: eine schematische Darstellung einer Draufsicht der in Figur 2 gezeigten modularen Trägereinheit;
- Fig. 4: eine schematische Darstellung einer Moduleinheit gemäß einer bevorzugten Ausführungsform;
- Fig. 5: eine schematische dreidimensionale Darstellung einer als Kulturgefäßmodul ausgebildeten modularen Trägereinheit gemäß einer weiteren bevorzugten Ausführungsform;
- Fig. 6: eine schematische Schnittansicht des in Figur 5 gezeigten Kulturgefäßmoduls;
- Fig. 7: eine schematische dreidimensionale Darstellung einer als Antriebsmodul ausgebildeten modularen Trägereinheit gemäß einer bevorzugten Ausführungsform;
- Fig. 8: eine schematische dreidimensionale Darstellung einer Moduleinheit gemäß einer weiteren bevorzugten Ausführungsform;
- Fig. 9: eine schematische Draufsicht der in Figur 8 gezeigten Moduleinheit;
- Fig. 10: eine schematische dreidimensionale Darstellung einer Moduleinheit gemäß einer weiteren bevorzugten Ausführungsform; und
- Fig. 11: eine schematische dreidimensionale Darstellung einer modularen Trägereinheit gemäß einer bevorzugten Ausführungsform.

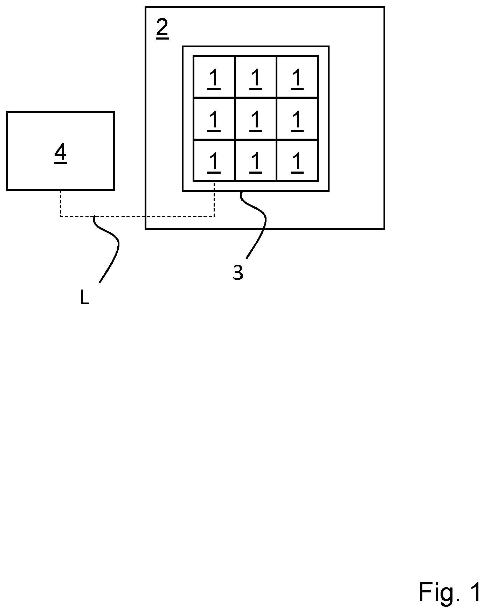

Figur 1 zeigt eine schematische Darstellung eines Inkubators 2 gemäß einer bevorzugten Ausführungsform. Dieser Inkubator kann in bevorzugter Weise zur Kultivierung dreidimensionaler Zellaggregate verwendet werden. Hierzu kann der Inkubator insbesondere mit einer Temperatur von bis zu 50°C und mit einer relativen Luftfeuchtigkeit von bis zu 99 % mit CO₂- und/oder N₂- und/oder O₂-Begasung betrieben werden.

In der vorliegenden Ausführungsform weist der Inkubator 2 eine Moduleinheit 3 und eine Steuerungseinheit 4 auf. Die Steuerungseinheit 4 ist mit der Moduleinheit 3 signaltechnisch und steuerungstechnisch gekoppelt. Vorliegend erfolgt die Kopplung der Steuerungseinheit 4 und der Moduleinheit 3 kabelgebunden über eine Leitung L. Es ist allerdings auch denkbar, die Steuerungseinheit 4 mit der Moduleinheit 3 drahtlos zu koppeln. Mittels der Steuerungseinheit kann die Kultivierung dreidimensionaler Zellaggregate gesteuert oder geregelt werden.

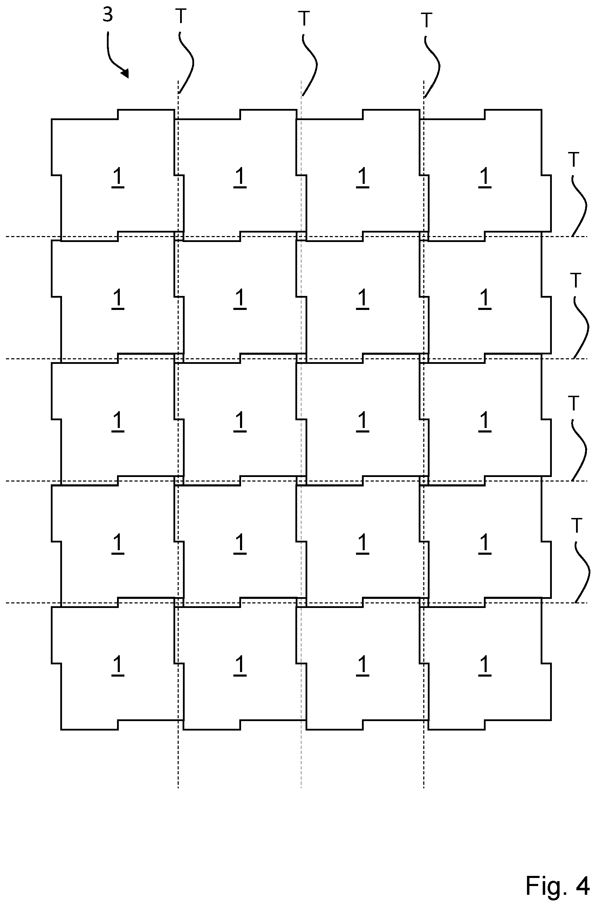

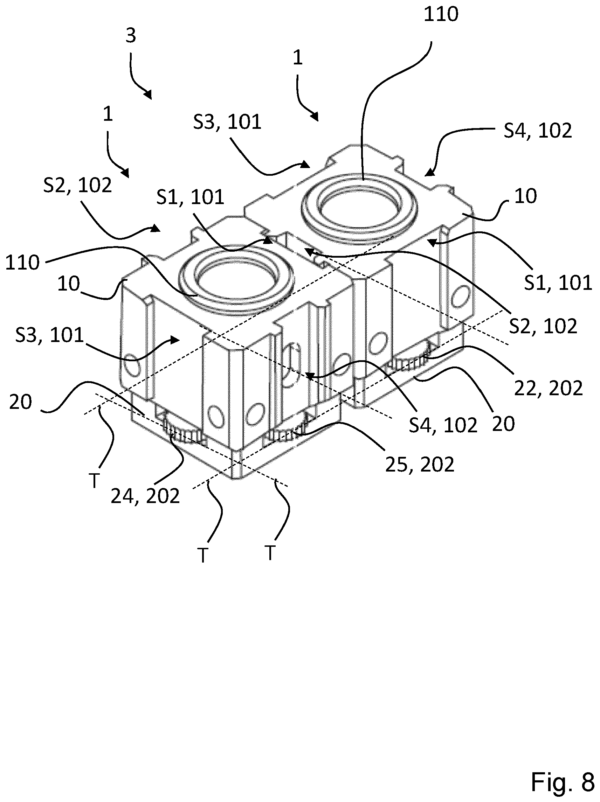

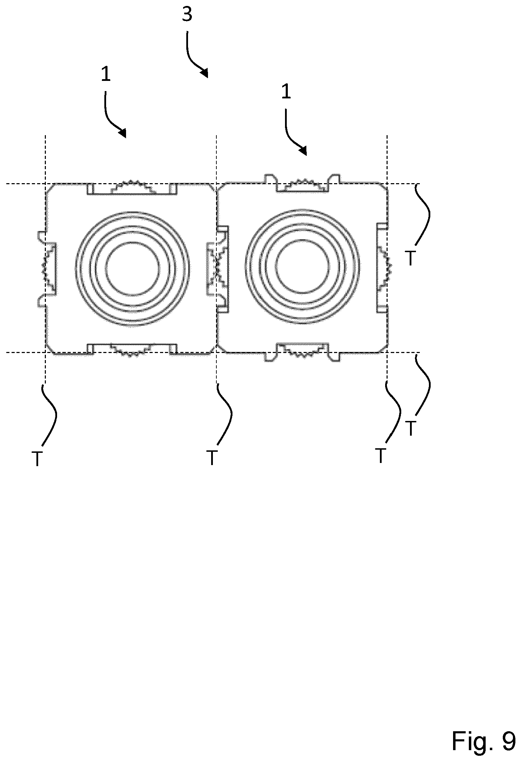

Die in Figur 1 schematisch dargestellte Moduleinheit wird in dieser Ausführungsform durch neun miteinander gekoppelte modulare Trägereinheiten 1 gebildet. Aufgrund des modularen Charakters der modularen Trägereinheit kann ein Benutzer die Moduleinheit individuell, je nach Kapazitätsbedarf aus einer Vielzahl modularer Trägereinheiten zusammenstellen. In Figur 4 ist beispielsweise eine Ausführungsform einer Moduleinheit 3 gezeigt, welche zwanzig modulare Trägereinheiten 1 umfasst. Figuren 8 und 9 zeigen wiederum eine bevorzugte Ausführungsform einer Moduleinheit 3, welche zwei modulare Trägereinheiten 1 umfasst. Es ist zu verstehen, dass diese Moduleinheiten 3 lediglich mögliche Beispiele sind und Moduleinheiten mit einer davon abweichenden Anzahl modularer Trägereinheiten 1 denkbar sind.

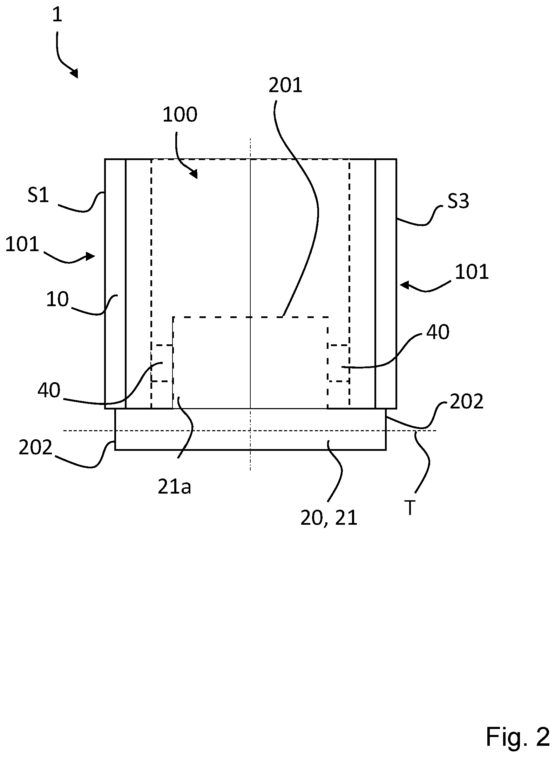

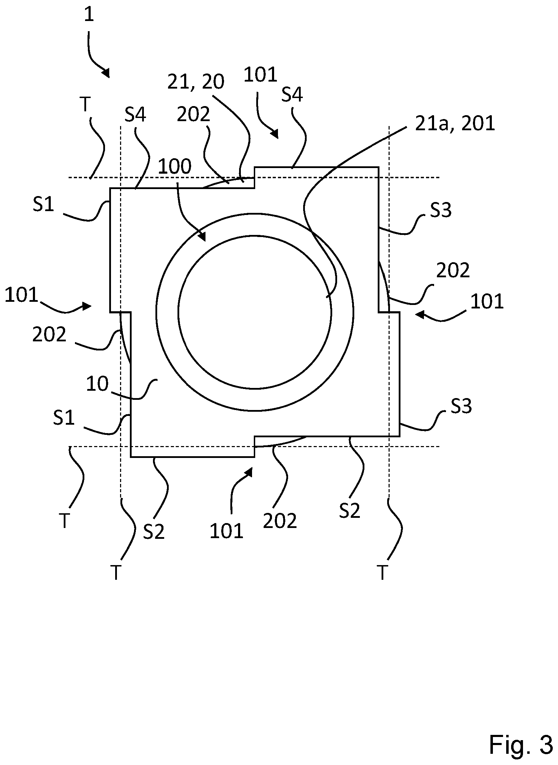

Eine modulare Trägereinheiten 1 ist in einer solchen Weise ausgestaltet, dass sie mit einer oder mehreren weiteren modularen Trägereinheiten 1 gekoppelt werden kann. Eine mögliche bevorzugte Ausführungsform der modularen Trägereinheit 1 ist in den Figuren 2 und 3 dargestellt. Figur 2 zeigt diese Ausführungsform der modularen Trägereinheiten 1 in einer schematischen Seitenansicht und Figur 3 in einer schematischen Draufsicht.

Hierzu weist die modulare Trägereinheiten 1 einen Trägerkörper 10 auf. Der Trägerkörper 10 ist in der vorliegenden Ausführungsform im Wesentlichen quaderförmig ausgebildet und weist vier Seitenflächen S1, S2, S3, S4 auf. Die vier Seitenflächen S1, S2, S3, S4 weisen jeweils eine erste Verbindungsschnittstelle 101 auf. Über die jeweilige erste Verbindungsschnittstelle 101 kann die modulare Trägereinheiten 1 mit einer weiteren modulare Trägereinheit 1 verbunden werden, so dass diese eine Moduleinheit 3 bilden, wie sie in Figur 4 dargestellt ist. In dieser bevorzugten Ausführungsform weisen alle modularen Trägereinheiten 1 die erste Verbindungsschnittstelle 101 auf. Die modularen Trägereinheiten 1 sind also jeweils über die erste Verbindungsschnittstelle 101 miteinander verbunden.

Zur Verbindung der modularen Trägereinheiten 1 miteinander ist die erste Verbindungsschnittstelle 101 vorliegend als formschlüssige Verbindung ausgebildet. Hierzu weist die erste Verbindungsschnittstelle 101 jeweils einen Vorsprung und eine Vertiefung aus. In der vorliegenden Ausführungsform ist die erste Verbindungsschnittstelle 101 bzw. sind die jeweiligen Seitenflächen S1, S2, S3, S4 stufenförmig ausgebildet. Bei einer Verbindung von zwei modularen Trägereinheiten 1 greift somit ein Vorsprung der ersten Verbindungsschnittstelle 101 an einer der vier Seitenfläche S1, S2, S3, S4 der einen modularen Trägereinheiten 1 in eine Vertiefung der ersten Verbindungsschnittstelle 101 an einer der vier Seitenfläche S1, S2, S3, S4 der anderen modularen Trägereinheiten 1. Dies geht ebenfalls aus der in Figur 4 schematisch dargestellten Moduleinheit 3 hervor.

Darüber hinaus weist der Trägerkörper eine Aufnahmeöffnung 100 auf. In dieser Aufnahmeöffnung 100 können zur Kultivierung der dreidimensionalen Zellaggregate Kulturgefäße angeordnet werden, in welchen die Zellaggregate kultiviert werden können. Eine modulare Trägereinheit 1, in der bzw. an der ein Kulturgefäß 5 angeordnet ist, wird vorliegend auch Kulturgefäßmodul genannt. Alternativ wird in der Aufnahmeöffnung 100 eine Antriebseinheit 6 angeordnet. Eine modulare Trägereinheit 1, in der bzw. an der eine Antriebseinheit 6 angeordnet ist, wird vorliegend auch Antriebsmodul genannt. Das Antriebsmodul dient dazu, Kulturgefäße 5, die in einem Kulturgefäßmodul angeordnet und mit dem Antriebsmodul gekoppelt sind, zu bewegen.

Mit dem Antriebsmodul lassen sich insbesondere eine Drehgeschwindigkeit, eine Drehrichtung, ein Drehwinkel und eine Drehbeschleunigung einstellen. Hierzu ist das Antriebsmodul, insbesondere die Antriebseinheit 6, mit der Steuerungseinheit 4 signaltechnisch gekoppelt. Vorzugsweise überschreitet die Drehgeschwindigkeit nicht die die Drehzahl von 153 Umdrehungen pro Minute. Ferner ist es bevorzugt, dass der Drehwinkel in einem Bereich vom 10° und 2550° liegt. Vorzugsweise überschreitet die Drehbeschleunigung nicht die Beschleunigung von 255 N/s²*10⁻⁶.

Zur Übertragung der Antriebsleitung der Antriebseinheit 6 auf Kulturgefäße 5 weisen die modularen Trägereinheiten jeweils eine Antriebsübertragungseinheit 20 auf. Diese Antriebsübertragungseinheit 20 ist dazu ausgebildet, ein Antriebsdrehmoment zu übertragen. Hierzu ist die Antriebsübertragungseinheit 20 gegenüber dem Trägerkörper 10 mittels einer Lagereinheit 40 drehbar gelagert. Ferner weist die Antriebsübertragungseinheit 20 in der vorliegenden Ausführungsform eine erste Antriebsübertragungsschnittstelle 201 auf. Diese erste Antriebsübertragungsschnittstelle 201 ist mit dem Kulturgefäß 5 oder der Antriebseinheit 6 koppelbar. Ferner weist die Antriebsübertragungseinheit 20 in der vorliegenden Ausführungsform eine zweite Antriebsübertragungsschnittstelle 202 auf. Diese zweite Antriebsübertragungsschnittstelle 202 ist mit einer zweiten Antriebsübertragungsschnittstelle 202 einer Antriebsübertragungseinheit 20 einer weiteren modularen Trägereinheit 1 oder der Antriebseinheit 6 koppelbar.

In der vorliegenden Ausführungsform umfasst die Antriebsübertragungseinheit 20 ein Zahnradgetriebe mit einem auf einer Welle 21a angeordneten Zahnrad 21. Die Welle 21a ist gegenüber dem Trägerkörper 10 mittels der Lagerung 40 drehbar gelagert. In dem vorliegenden Ausführungsbeispiel bildet die Welle 21a an einem Ende, welches der Aufnahmeöffnung zugewandt ist, die erste Antriebsübertragungsschnittstelle 201 aus. Entsprechend bildet das Zahnrad 21 die zweite Antriebsübertragungsschnittstellen 202 aus.

Der Durchmesser des Zahnrads 21 ist hierbei in einer solchen Weise gewählt, dass bei einer Verbindung von zwei modularen Trägereinheiten 1 die Zahnräder 21 der modularen Trägereinheiten 1 zur Übertragung eines Drehmoments bzw. einer Drehbewegung ineinandergreifen. Somit muss die zweite Antriebsübertragungsschnittstelle 202 des Zahnrads 21 der jeweiligen modularen Trägereinheit in einer Ebene liegen, die einer Verbindungsebene entspricht, welche durch die jeweiligen Verbindungsschnittstellen an den Seitenflächen S1, S2, S3, S4 definiert wird. Dies erfordert, dass der Teilkreisdurchmesser des Zahnrads 21 in einer solchen Weise gewählt wird, dass der Kontaktpunkt zwischen den Zahnrädern 21 von zwei zu verbindenden modularen Trägereinheiten 1 derart gewählt wird, dass eine Tangente T der Teilkreisdurchmesser in diesem Kontaktpunkt sich parallel zu den jeweiligen Seitenflächen der Trägerkörper bzw. in den durch die an den Seitenflächen ausgebildeten Verbindungsschnittstellen definierten Verbindungsebenen liegen. Hierbei ist nach Erkenntnis der Erfinder insbesondere zu beachten, dass die Zahnräder denselben Durchmesser und dieselbe Zahnzahl aufweisen. Hierdurch wird sichergestellt, dass sich die Drehzahl nicht ändert und alle modularen Trägereinheiten dieselbe Drehzahl aufweisen. Es kann nach Erkenntnis der Erfinder auch bevorzugt sein, Zahnräder unterschiedlicher Durchmesser bereitzustellen, um Zellen unter verschiedenen Bedingungen zu kultivieren.

Bei den modularen Trägereinheiten 1 der in Figur 4 gezeigten Moduleinheit 3 sind die in den äußeren Ecken angeordneten modularen Trägereinheiten 1 über die zweite Antriebsübertragungsschnittstelle 202 mit zweiten Antriebsübertragungsschnittstellen 202 von zwei modularen Trägereinheiten 1 verbunden. Die modularen Trägereinheiten, die am Rand zwischen den an den äußeren Ecken angeordneten modularen Trägereinheiten angeordnet sind, sind über die zweite Antriebsübertragungsschnittstelle 202 mit zweiten Antriebsübertragungsschnittstellen 202 von drei modularen Trägereinheiten 1 verbunden. Die sechs in der Mitte der Moduleinheit 3 angeordneten modularen Trägereinheiten 1 sind über die zweite Antriebsübertragungsschnittstelle 202 mit zweiten Antriebsübertragungsschnittstellen 202 von vier modularen Trägereinheiten 1 verbunden.

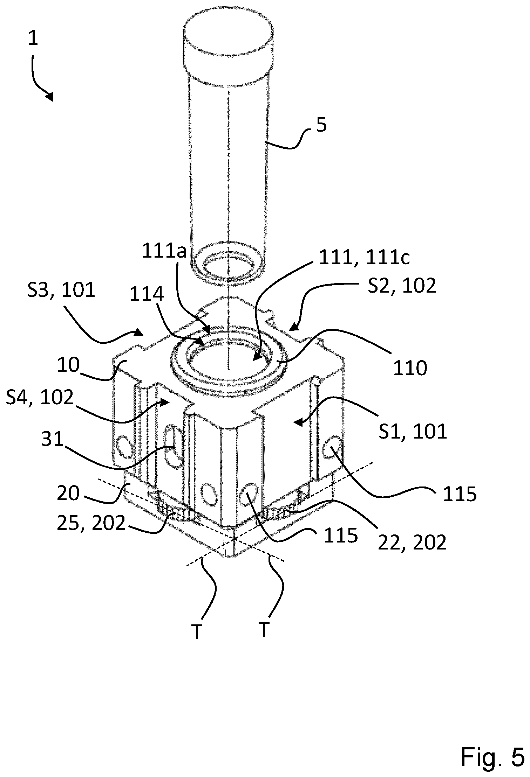

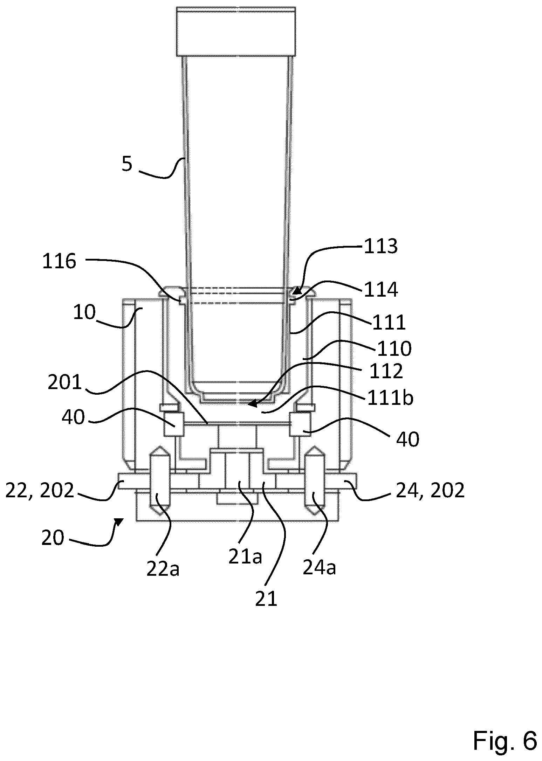

Figur 5 ist eine schematische, dreidimensionale Darstellung einer als Kulturgefäßmodul ausgebildeten modularen Trägereinheit 1 gemäß einer weiteren bevorzugten Ausführungsform. Figur 6 ist eine schematische Schnittansicht des in Figur 6 gezeigten Kulturgefäßmoduls. In dieser Ausführungsform ist der Trägerkörper im Wesentlichen würfelförmig ausgebildet. Entsprechend weist der Trägerkörper 10 ebenfalls vier Seitenflächen S1, S2, S3, S4 auf. In dieser Ausführungsform sind jedoch zwei verschiedene Verbindungsschnittstellen vorgesehen. Eine erste Verbindungsschnittstelle 101 ist als eine Art Nut vorgesehen und eine zweite Verbindungsschnittstelle 102 ist durch zwei sich parallel erstreckende leistenartige Vorsprünge ausgebildet. Vorzugsweise weisen sowohl die erste Verbindungsschnittstelle 101 als auch die zweite Verbindungsschnittstelle 102 für eine kraftschlüssige Verbindung Magnete 115 auf. Es ist vorgesehen, dass zwei der vier Seitenflächen S1, S2, S3, S4 die erste Verbindungsschnittstelle aufweisen und die zwei anderen der vier Seitenflächen S1, S2, S3, S4 die zweite Verbindungsschnittstelle aufweisen. In dieser bevorzugten Ausführungsform weisen jeweils zwei sich gegenüberliegend erstreckende Seitenflächen S1, S2, S3, S4 dieselbe Verbindungsschnittstelle 101, 102 auf. Vorliegend weisen somit die erste und dritte Seitenfläche S1, S3 die erste Verbindungsschnittstelle 101 und die zweite und vierte Seitenfläche S2, S4 die zweite Verbindungsschnittstelle 102 auf.

In dieser Ausführungsform ist ferner eine topfförmige Kulturgefäßaufnahmeeinheit 110 vorgesehen. Diese Kulturgefäßaufnahmeeinheit 110 ist drehbar in der Aufnahmeöffnung 100 angeordnet. Die Kulturgefäßaufnahmeeinheit 110 ist dazu ausgebildet, das Kulturgefäß aufzunehmen und die Drehbewegung von der Antriebsübertragungseinheit 20 auf die Kulturgefäßaufnahmeeinheit 110 zu übertragen. Hierzu ist die Kulturgefäßaufnahmeeinheit 110 mit der ersten Antriebsübertragungsschnittstelle 201 gekoppelt. Die Kulturgefäßaufnahmeeinheit 110 weist zur Aufnahme des Kulturgefäßes 5 ein Sackloch 111 auf. Zur Befestigung des Kulturgefäßes in der Kulturgefäßaufnahmeeinheit 110 ist ein erster Befestigungsabschnitt 112 und ein zweiter Befestigungsabschnitt 113 vorgesehen. In der vorliegenden Ausführungsform befindet sich der erste Befestigungsabschnitt 112 am Boden 111b bzw. im Bereich des Bodens 111b des Sacklochs. Der erste Befestigungsabschnitt 112 ermöglicht eine formschlüssige Verbindung des Kulturgefäßes 5 in der Kulturgefäßaufnahmeeinheit 110. Hierzu kann der erste Befestigungsabschnitt 112 beispielsweise Vorsprünge aufweisen, die in entsprechende Vertiefungen an der Unterseite des Kulturgefäßes greifen. Umgekehrt ist es selbstverständlich denkbar, dass der erste Befestigungsabschnitt 112 Vertiefungen aufweist, in die entsprechende Vorsprünge an der Unterseite des Kulturgefäßes greifen.

Der zweite Befestigungsabschnitt 113 ist am Bereich der Öffnung 111a des Sacklochs 111 vorgesehen. Der zweite Befestigungsabschnitt ermöglicht in der vorliegenden Ausführungsform eine kraftschlüssige Verbindung zwischen dem Kulturgefäß 5 und der Kulturgefäßaufnahmeeinheit 110. Hierzu ist ein Gummiring als elastisches Befestigungselement 114 vorgesehen, welches in einer umlaufenden Nut 116, die im Bereich der Öffnung 111a des Sacklochs 111 eingelassen ist, eingesetzt wird.

Die in den Figuren 5 bis 8 dargestellte Ausführungsform einer modularen Trägereinheit 1 umfasst als Antriebsübertragungseinheit 20 ebenfalls ein Zahnradgetriebe. In dieser Ausführungsform weist das Zahnradgetriebe hingegen eine Zentralradwelle 21a und ein auf der Zentralradwelle 21a angeordnetes Zentral-Zahnrad 21 auf. Die Zentralradwelle 21a ist gegenüber dem Trägerkörper 10 mittels einer Lagerung 40 drehbar gelagert und bildet an einem Ende die erste Antriebsübertragungsschnittstelle 201 aus. Vorliegend ist nun je Seitenfläche S1, S2, S3, S4 ein Außen-Zahnrad 22, 23, 24, 25 vorgesehen, das in das Zentral-Zahnrad 21 greift. Die Außen-Zahnräder 22, 23, 24, 25 sind jeweils mittels eines Außenradzapfens 22a, 23a, 24a, 25a gegenüber dem Trägerkörper 10 drehbar gelagert. In diesem Ausführungsbeispiel bilden die Außen-Zahnräder 22, 23, 24, 25 jeweils Antriebsübertragungsschnittstellen 202.

Die Durchmesser des Zentral-Zahnrads 21 und der Außen-Zahnräder 22, 23, 24, 25 sind hierbei in einer solchen Weise gewählt, dass bei einer Verbindung von zwei modularen Trägereinheiten 1 die jeweiligen Außen-Zahnräder 22, 23, 24, 25 der modularen Trägereinheiten 1 zur Übertragung eines Drehmoments bzw. einer Drehbewegung ineinandergreifen. Somit muss die zweite Antriebsübertragungsschnittstellen 202 der Außen-Zahnräder 22, 23, 24, 25 der jeweiligen modularen Trägereinheit in einer Ebene liegen, die einer Verbindungsebene entspricht, welche durch die jeweiligen Verbindungsschnittstellen an den Seitenflächen S1, S2, S3, S4 definiert wird. Dies erfordert, dass der Teilkreisdurchmesser der Außen-Zahnräder 22, 23, 24, 25 in einer solchen Weise gewählt wird, dass der Kontaktpunkt zwischen den Außen-Zahnrädern 22, 23, 24, 25 von zwei zu verbindenden modularen Trägereinheiten 1 derart gewählt wird, dass eine Tangente T der Teilkreisdurchmesser in diesem Kontaktpunkt sich parallel zu den jeweiligen Seitenflächen der Trägerkörper erstreckt bzw. in den durch die an den Seitenflächen ausgebildeten Verbindungsschnittstellen definierten Verbindungsebenen liegt. Hierbei ist nach Erkenntnis der Erfinder zu beachten, dass die Zahnräder denselben Durchmesser und dieselbe Zahnzahl aufweisen. Dies stellt sicher, dass sich die Drehzahl nicht ändert und alle modularen Trägereinheiten dieselbe Drehzahl aufweisen. Es kann nach Erkenntnis der Erfinder auch bevorzugt sein, Zahnräder unterschiedlicher Durchmesser bereitzustellen, um Zellen unter verschiedenen Bedingungen zu kultivieren.

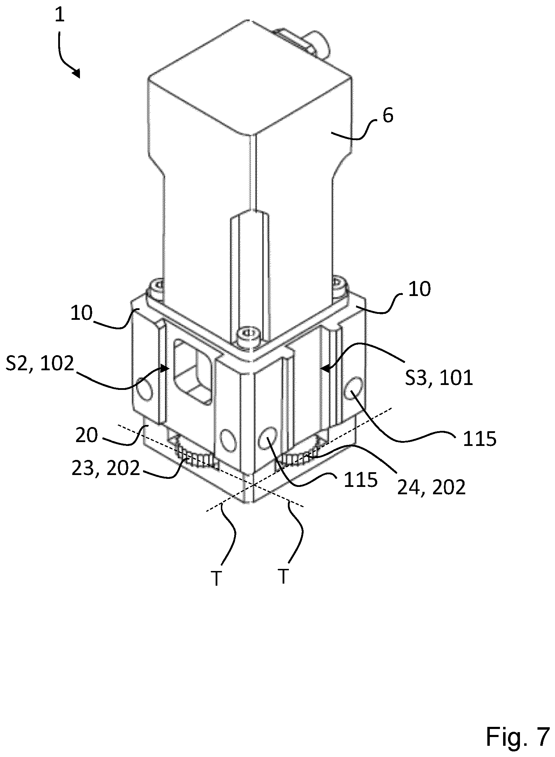

Die in Figur 7 schematisch dargestellte modulare Trägereinheit 1 ist hinsichtlich des Trägerkörpers 10 und der Antriebsübertragungseinheit 20 analog zu dem Trägerkörper 10 und der Antriebsübertragungseinheit 20 der in den Figuren 5 und 6 dargestellten modularen Trägereinheit 1 ausgebildet. Im Unterschied zu der in den Figuren 5 bis 6 gezeigten Ausführungsform der modularen Trägereinheit 1 weist die in Figur 7 dargestellte modulare Trägereinheit keine Kulturgefäßaufnahmeeinheit 110 auf. Stattdessen ist ein Elektromotor als Antriebseinheit 6 vorgesehen, der in der Aufnahmeöffnung 100 mittels Schrauben an dem Trägerkörper 10 befestigt ist. Zur Übertragung der Drehbewegung von der Antriebseinheit 6 auf die Antriebsübertragungseinheit 20, ist die Antriebseinheit 6 entsprechend mit der ersten Antriebsübertragungsschnittstelle 201 gekoppelt.

Figur 8 ist eine schematische, dreidimensionale Darstellung einer Moduleinheit 3 gemäß einer weiteren bevorzugten Ausführungsform. Figur 9 ist eine schematische Draufsicht der in Figur 8 gezeigten Moduleinheit 3. Die in den Figuren 8 und 9 gezeigte Moduleinheit 3, ist aus modularen Trägereinheiten 1 zusammengesetzt, die gemäß der in den Figuren 5 und 6 gezeigten Ausführungsform ausgebildet sind. In den Figuren 8 und 9 sind die modularen Trägereinheiten 1 ohne Kulturgefäße 5 abgebildet.

Die in den Figuren 8 und 9 dargestellte Moduleinheit 3 umfasst zwei miteinander verbundene modulare Trägereinheiten 1. In diesem Fall liegt die eine modulare Trägereinheit 1 mit der ersten Seitenfläche 1 an der zweiten Seitenfläche S2 der anderen modularen Trägereinheit 1 an. Durch die nutenförmige Ausgestaltung der ersten Verbindungsschnittstelle 101 an der ersten Seitenfläche S1 und die leistenförmigen Vorsprünge der zweiten Verbindungsschnittstelle 102 sind die beiden modularen Trägereinheiten 1 formschlüssig miteinander verbunden. Zusätzlich weisen sowohl die erste als auch die zweite Verbindungsschnittstelle 101, 102 korrespondierend angeordnete und ausgebildete Magnete 115 auf, die zwischen den beiden modularen Trägereinheiten 1 eine kraftschlüssige Verbindung herstellen. Insbesondere aus der Draufsicht auf die Moduleinheit 3 in Figur 9 geht hervor, dass die Durchmesser des Zentral-Zahnrads 21 und der Außen-Zahnräder 22, 23, 24, 25 in einer solchen Weise gewählt sind, dass bei der Verbindung der beiden modularen Trägereinheiten 1 die jeweiligen Außen-Zahnräder 22, 23, 24, 25 der modularen Trägereinheiten 1 zur Übertragung eines Drehmoments bzw. einer Drehbewegung ineinandergreifen. Hierzu liegen die zweiten Antriebsübertragungsschnittstellen 202 der Außen-Zahnräder 22, 23, 24, 25 der jeweiligen modularen Trägereinheit in einer Ebene, die der Verbindungsebene entspricht, welche durch die jeweiligen Verbindungsschnittstellen an den Seitenflächen S1, S2, S3, S4 definiert wird. Entsprechend ist der Teilkreisdurchmesser der Außen-Zahnräder 22, 23, 24, 25 in einer solchen Weise gewählt, dass der Kontaktpunkt zwischen den Außen-Zahnrädern 22, 23, 24, 25 von zwei zu verbindenden modularen Trägereinheiten 1 derart gewählt ist, dass die Tangente T der Teilkreisdurchmesser sich in diesem Kontaktpunkt parallel zu den jeweiligen Seitenflächen S1, S2, S3, S4 der Trägerkörper 10 erstreckt bzw. in den durch die an den Seitenflächen S1, S2, S3, S4 ausgebildeten Verbindungsschnittstellen 101, 102 definierten Verbindungsebenen liegt.

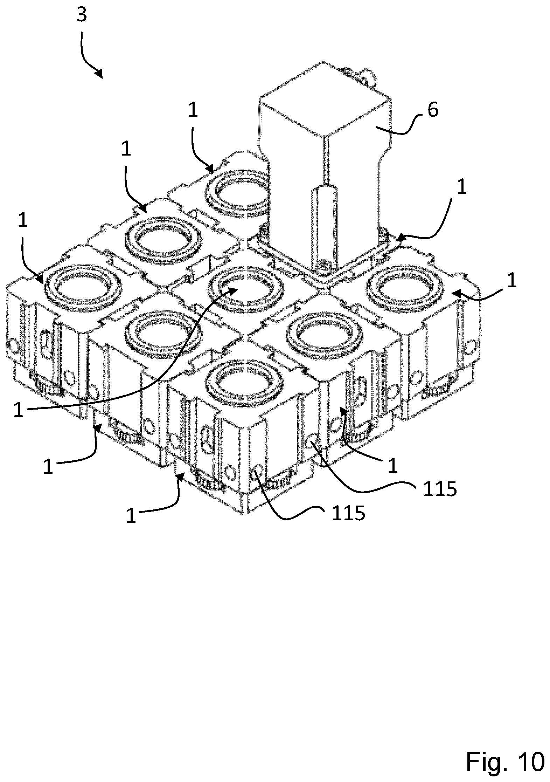

Figur 10 ist eine schematische, dreidimensionale Darstellung einer Moduleinheit 3 gemäß einer weiteren bevorzugten Ausführungsform. In diesem Ausführungsbeispiel sind neun modulare Trägereinheiten vorgesehen. Acht der neun in Figur 10 gezeigten modularen Trägereinheiten 1 entsprechend der in den Figuren 5 und 6 gezeigten modularen Trägereinheit 1. Eine der neun in Figur 10 gezeigten modularen Trägereinheiten 1 entspricht der in Figur 7 gezeigten modularen Trägereinheit 1. Die modulare Trägereinheit 1 mit der Antriebseinheit 6 treibt über die Zahnradgetriebe der jeweiligen modularen Trägereinheiten und die mit den Zahnradgetrieben gekoppelten Kulturgefäßaufnahmeeinheiten 110 an. In Figur 10 ist die Moduleinheit 3 ohne die Kulturgefäße 5 gezeigt. Diese können jedoch zur Kultivierung von dreidimensionalen Zellaggregaten in die Kulturgefäßaufnahmeeinheiten 110 in einem Inkubator 2 eingesetzt werden.

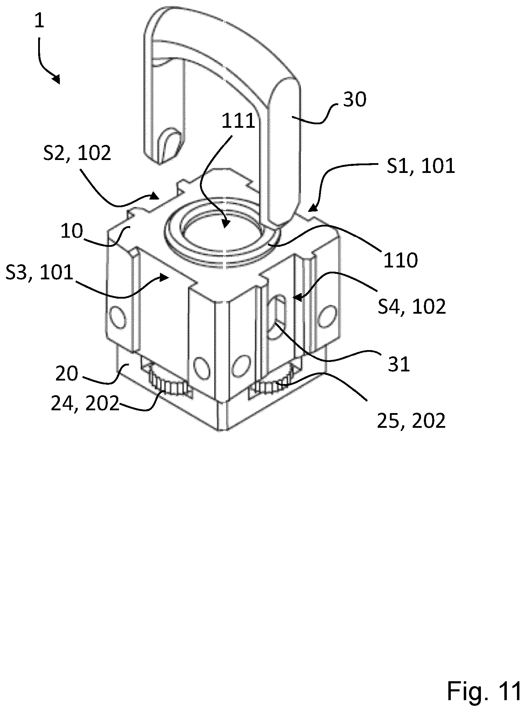

Figur 11 ist eine schematische, dreidimensionale Darstellung einer modularen Trägereinheit 1 gemäß einer weiteren bevorzugten Ausführungsform. Die in Figur 11 dargestellte modulare Trägereinheit 1 entspricht der modularen Trägereinheit 1, wie diese beispielsweise hinsichtlich der Figuren 5 und 6 zuvor beschrieben worden ist. In dieser bevorzugten Ausführungsform ist nun ein Demontagegriff 30 vorgesehen, der zur Demontage einer modularen Trägereinheit ausgebildet ist. Hierzu weist der Trägerkörper einen Griffbefestigungsabschnitt 31 auf, der für eine form- und kraftschlüssige Verbindung mit dem Demontagegriff ausgebildet ist. In der vorliegenden Ausführungsform weist der Griffbefestigungsabschnitt 31 eine Nut auf, in welche ein Schnapphaken des Demontagegriffs einrasten kann. Der Griffbefestigungsabschnitt 31 ist hierzu an zwei gegenüberliegenden Seitenflächen ausgebildet. In der in Figur 11 dargestellten Ausführungsform weist die vierte Seitenfläche S4 im Griffbefestigungsabschnitt 31 eine entsprechende Nut auf. Die zweite Seitenfläche S4 weist einen entsprechenden Griffbefestigungsabschnitt 31 mit Nut auf, die in der gewählten Darstellung der modularen Trägereinheit 1 in Figur 11 durch den Trägerkörper 10 verdeckt wird. Zur Demontage wird das Kulturgefäß zunächst der modularen Trägereinheit 1 entnommen. Anschließend wird der Demontagegriff 30 auf den Trägerkörper aufgeschoben, so dass die einen Schnapphaken ausbildenden seitlichen Arme in die Nut des Griffbefestigungsabschnitts 31 einrasten. Die modulare Trägereinheit 1 kann dann mit Hilfe des Demontagegriffs 30 von den anderen modularen Trägereinheiten 1 und somit aus einer Moduleinheit 3 herausgelöst werden. Die Montage der modularen Trägereinheit 1 erfolgt analog, in umgekehrter Reihenfolge. Der Demontagegriff kann somit auch zur Montage der modularen Trägereinheit 1 genutzt werden.

### Bezugszeichenliste

- 1: Modulare Trägereinheit
- 2: Inkubator
- 3: Moduleinheit
- 4: Steuerungseinheit
- 5: Kulturgefäß
- 6: Antriebseinheit
- 10: Trägerkörper
- 100: Aufnahmeöffnung
- 101: erste Verbindungsschnittstelle
- 102: zweite Verbindungsschnittstelle
- 110: Kulturgefäßaufnahmeeinheit
- 111: Sackloch
- 111a: Öffnung des Sacklochs
- 111b: Boden des Sacklochs
- 111c: Seitenwand des Sacklochs
- 112: erster Befestigungsabschnitt
- 113: zweiter Befestigungsabschnitt
- 114: Befestigungselement
- 115: Magnet
- 116: umlaufende Nut
- 20: Antriebsübertragungseinheit
- 21: Zahnrad
- 21a: Welle des Zahnrads
- 22, 23, 24, 25: Außen-Zahnrad
- 22a, 23a, 24a, 25a: Außenradzapfen des Außen-Zahnrads
- 201: erste Antriebsübertragungsschnittstelle
- 202: zweite Antriebsübertragungsschnittstelle
- 30: Demontagegriff
- 31: Griffbefestigungsabschnitt
- L: Leitung
- S1, S2, S3, S4: Seitenflächen
- T: Tangente

## Patentansprüche

1. Modulare Trägereinheit (1) für einen Inkubator (2), insbesondere für eine Moduleinheit (3) eines Inkubators (2), umfassend:
- einen Trägerkörper (10) aufweisend
o eine Aufnahmeöffnung (100), die zur Aufnahme eines Kulturgefäßes (5) oder einer Antriebseinheit (6) ausgebildet ist, und
o drei, vier oder mehr Seitenflächen (S1, S2, S3, S4),
o wobei mindestens eine der Seitenflächen (S1, S2, S3, S4) eine erste Verbindungsschnittstelle (101) aufweist, die zur Verbindung der modularen Trägereinheit (1) mit einer weiteren modularen Trägereinheit (1) ausgebildet ist,
- eine Antriebsübertragungseinheit (20), die zur Übertragung eines Antriebsdrehmoments ausgebildet ist und an dem Trägerkörper (10) angeordnet ist, die Antriebsübertragungseinheit (20) aufweisend:
o mindestens eine erste Antriebsübertragungsschnittstelle (201), welche mit dem Kulturgefäß (5) oder der Antriebseinheit (6) koppelbar ist,
o eine zweite Antriebsübertragungsschnittstelle (202), die mit einer zweiten Antriebsübertragungsschnittstelle (202) einer Antriebsübertragungseinheit (20) der weiteren modularen Trägereinheit (1) oder der Antriebseinheit (6) koppelbar ist.

2. Modulare Trägereinheit (1) nach dem vorhergehenden Anspruch 1, aufweisend die Antriebseinheit (6), die an und/oder in der Aufnahmeöffnung (100) an dem Trägerkörper (10) befestigt ist und zur Übertragung der Drehbewegung von der Antriebseinheit (6) auf die Antriebsübertragungseinheit (20) mit der ersten Antriebsübertragungsschnittstelle (201) gekoppelt ist,
wobei die Antriebseinheit (6) vorzugsweise ein Elektromotor, vorzugsweise ein Schrittmotor ist.

3. Modulare Trägereinheit (1) nach dem vorhergehenden Anspruch 1, aufweisend eine drehbar in der Aufnahmeöffnung (100) angeordnete Kulturgefäßaufnahmeeinheit (110), die zur Aufnahme des Kulturgefäßes (5) ausgebildet ist und zur Übertragung der Drehbewegung von der Antriebsübertragungseinheit (20) auf die Kulturgefäßaufnahmeeinheit (110) mit der ersten Antriebsübertragungsschnittstelle (201) gekoppelt ist,
wobei vorzugsweise in der Kulturgefäßaufnahmeeinheit (110) eine oder mehrere Kulturgefäße (5) anordenbar oder angeordnet sind.

4. Modulare Trägereinheit (1) nach dem vorhergehenden Anspruch 3, wobei die Kulturgefäßaufnahmeeinheit (110) zur Aufnahme des Kulturgefäßes (5)
- topfförmig ausgebildet ist und/oder
- kegelförmig ausgebildet ist und/oder
- ein Sackloch (111) aufweist, das sich zwischen einer Öffnung (111a) und einem Boden (111b) erstreckt.

5. Modulare Trägereinheit (1) nach dem vorhergehenden Anspruch 4, wobei das Sackloch (111)
- einen ersten Befestigungsabschnitt (112) aufweist, der zur kraft- und formschlüssigen Verbindung mit dem Kulturgefäß (5) ausgebildet ist; und/oder
- einen zweiten Befestigungsabschnitt (113) aufweist, der zur kraft- und formschlüssigen Verbindung mit dem Kulturgefäß (5) ausgebildet ist.

6. Modulare Trägereinheit (1) nach dem vorhergehenden Anspruch 5, wobei
- der erste Befestigungsabschnitt (112)
o am Boden (111b) und/oder im Bereich des Bodens (111b) des Sacklochs (111) ausgebildet ist, und/oder
o für eine formschlüssige Verbindung Vorsprünge und/oder Vertiefungen aufweist; und/oder
- der zweite Befestigungsabschnitt (113)
o am und/oder im Bereich der Öffnung (111a) ausgebildet ist, und/oder
o für eine kraftschlüssige Verbindung ein elastisches Befestigungselement (114), insbesondere einen Gummiring aufweist, wobei das Befestigungselement (114) vorzugsweise in einer Nut (116) angeordnet ist, die in einer Seitenwand (111c) des Sacklochs (111) ausgebildet wird.

7. Modulare Trägereinheit (1) nach einem der vorhergehenden Ansprüche 1 bis 6,
- wobei mindestens eine der Seitenflächen (S1, S2, S3, S4) eine zweite Verbindungsschnittstelle (102) aufweist, die zur Verbindung der modularen Trägereinheit (1) mit einer weiteren modularen Trägereinheit (1) ausgebildet ist, und/oder
- wobei die erste Verbindungsschnittstelle (101) und/oder die zweite Verbindungsschnittstelle (102) für eine formschlüssige und/oder kraftschlüssige Verbindung ausgebildet ist.

8. Modulare Trägereinheit (1) nach einem der vorhergehenden Ansprüche 1 bis 7, wobei
- die erste Verbindungsschnittstelle (101) von der zweiten Verbindungsschnittstelle (102) verschieden ausgebildet ist, oder
- die erste Verbindungsschnittstelle (101) der zweiten Verbindungsschnittstelle (102) entspricht.

9. Modulare Trägereinheit (1) nach einem der vorhergehenden Ansprüche 1 bis 8, wobei
- die erste Verbindungsschnittstelle (101) zur Verbindung mit einer zweiten Verbindungsschnittstelle (102) der weiteren Trägereinheit (1) ausgebildet ist und/oder
- die zweite Verbindungsschnittstelle (102) zur Verbindung mit einer ersten Verbindungsschnittstelle (101) der weiteren Trägereinheit (1) ausgebildet ist.

10. Modulare Trägereinheit (1) nach einem der vorhergehenden Ansprüche 1 bis 9, wobei
- die erste Verbindungsschnittstelle (101) zur Verbindung mit der weiteren Trägereinheit (1)
o für eine formschlüssige Verbindung Vorsprünge und/oder Vertiefungen aufweist und/oder
o für eine kraftschlüssige Verbindung einen Magnet (115) und/oder ein Federsystem aufweist, und/oder
- die zweite Verbindungsschnittstelle (102) zur Verbindung mit der weiteren Trägereinheit (1)
o für eine formschlüssige Verbindung Vorsprünge und/oder Vertiefungen aufweist und/oder
o für eine kraftschlüssige Verbindung einen Magnet (115) und/oder ein Federsystem aufweist.

11. Modulare Trägereinheit (1) nach einem der vorhergehenden Ansprüche 1 bis 10, aufweisend einen Demontagegriff (30), der mit dem Trägerkörper (10) zur Demontage einer mit einer weiteren modularen Trägereinheit (1) verbundenen modularen Trägereinheit (1) koppelbar ist, wobei der Trägerkörper (10) vorzugsweise einen Griffbefestigungsabschnitt (31) aufweist, der für eine form- und/oder kraftschlüssige Verbindung mit dem Demontagegriff (30) ausgebildet ist.

12. Modulare Trägereinheit (1) nach einem der vorhergehenden Ansprüche 1 bis 11,
- wobei der Trägerkörper (10) würfelförmig und/oder quaderförmig ausgebildet ist, und/oder
- wobei die Antriebsübertragungseinheit (20) gegenüber dem Trägerkörper (10) mittels einer Lagereinheit (40) drehbar gelagert ist,
- wobei die Antriebsübertragungseinheit (20) als Zahnradgetriebe mit mindestens einem Zahnrad (21) ausgebildet ist oder ein Zahnradgetriebe mit mindestens einem Zahnrad (21) umfasst,
- wobei die Antriebsübertragungseinheit (20) als Riemen- und/oder Kettentrieb ausgebildet ist oder einen Riemen- und/oder Kettentrieb umfasst,
- wobei die Antriebsübertragungseinheit (20) als elektromagnetischer Antrieb ausgebildet ist oder einen elektromagnetischen Antrieb umfasst.

13. Modulare Trägereinheit (1) nach einem der vorhergehenden Ansprüche 1 bis 12, wobei das Zahnradgetriebe genau ein auf einer Welle (21a) angeordnetes Zahnrad (21) aufweist, wobei
- die Welle (21a) gegenüber dem Trägerkörper (10) drehbar gelagert ist, und
- die Welle (21a) an einem Ende die erste Antriebsübertragungsschnittstelle (201) ausbildet, und
- das Zahnrad die zweiten Antriebsübertragungsschnittstellen (202) ausbildet.

14. Modulare Trägereinheit (1) nach einem der vorhergehenden Ansprüche 1 bis 13, wobei das Zahnradgetriebe
- ein auf einer Zentralradwelle (21a) angeordnetes Zentral-Zahnrad (21) aufweist und die Zentralradwelle (21a) an einem Ende die erste Antriebsübertragungsschnittstelle (201) ausbildet, wobei die Zentralradwelle (21a) gegenüber dem Trägerkörper (10) drehbar gelagert ist und
- je Seitenfläche (S1, S2, S3, S4) mindestens ein Außen-Zahnrad (22, 23, 24, 25) aufweist, das in das Zentral-Zahnrad (21) greift, wobei die Außen-Zahnräder (22, 23, 24, 25) jeweils mittels eines Außenradzapfens (22a, 23a, 24a, 25a) gegenüber dem Trägerkörper (10) drehbar gelagert sind.

15. Moduleinheit (3) aufweisend mindestens zwei modulare Trägereinheiten (1) nach einem der vorhergehenden Ansprüche 1 bis 14.

16. Moduleinheit (3) nach dem vorhergehenden Anspruch 15, wobei mindestens eine der mindestens zwei modularen Trägereinheiten (1) eine Antriebseinheit (6) gemäß Anspruch 2 aufweist und mindestens eine der mindestens zwei modularen Trägereinheiten (1) eine Kulturgefäßaufnahmeeinheit (110), insbesondere eine Kulturgefäßaufnahmeeinheit (110) mit einem Kulturgefäß (5), gemäß Anspruch 3 aufweist.

17. Inkubator (2) aufweisend mindestens eine modulare Trägereinheit (1) nach einem der vorhergehenden Ansprüche 1 bis 14 und/oder eine Moduleinheit (3) nach einem der vorhergehenden Ansprüche 15 oder 16,
der Inkubator (2) vorzugsweise aufweisend eine Steuerungseinheit (4) zur Steuerung der modularen Trägereinheit (1) und/oder der Moduleinheit (3), wobei die Steuerungseinheit (4) signaltechnisch und/oder steuerungstechnisch, insbesondere mittels einer Leitung (L) kabelgebunden oder drahtlos, mit der modularen Trägereinheit (1) und/oder der Moduleinheit (3) gekoppelt ist.

18. Verwendung der modularen Trägereinheit (1) nach einem der vorhergehenden Ansprüche 1 bis 14 und/oder der Moduleinheit (3) nach einem der vorhergehenden Ansprüche 15 oder 16 in einem Inkubator (2).

## Claims

1. Modular carrier unit (1) for an incubator (2), in particular for a module unit (3) of an incubator (2), comprising:
- a carrier body (10) comprising
o a receiving opening (100) designed to receive a culture vessel (5) or a drive unit (6), and
∘ three, four or more side surfaces (S1, S2, S3, S4),
∘ wherein at least one of the side surfaces (S1, S2, S3, S4) comprises a first connection interface (101) configured for connecting the modular carrier unit (1) to a further modular carrier unit (1),
- a drive transmission unit (20) configured to transmit drive torque and arranged on the carrier body (10), the drive transmission unit (20) comprising:
o at least one first drive transmission interface (201) which can be coupled to the culture vessel (5) or the drive unit (6),
o a second drive transmission interface (202) which can be coupled to a second drive transmission interface (202) of a drive transmission unit (20) of the further modular carrier unit (1) or the drive unit (6).

2. Modular carrier unit (1) according to the preceding claim 1, comprising the drive unit (6), which is secured to and/or within the receiving opening (100) on the carrier body (10) and is coupled to the first drive transmission interface (201) for transmitting the rotational movement from the drive unit (6) to the drive transmission unit (20),
wherein the drive unit (6) is preferably an electric motor, preferably a stepper motor.

3. **Modular** carrier unit (1) according to the preceding claim 1, comprising a culture vessel receiving unit (110) arranged rotatably in the receiving opening (100), which is designed to receive the culture vessel (5) and is coupled to the first drive transmission interface (201) for transmitting the rotational movement from the drive transmission unit (20) to the culture vessel receiving unit (110),
wherein preferably one or more culture vessels (5) can be arranged or are arranged in the culture vessel receiving unit (110).

4. Modular carrier unit (1) according to the preceding claim 3, wherein the culture vessel receiving unit (110) for receiving the culture vessel (5)
- is pot-shaped and/or
- is conical in shape and/or
- has a blind hole (111) extending between an opening (111a) and a bottom (111b).

5. Modular carrier unit (1) according to the preceding claim 4, wherein the blind hole (111)
- comprises a first fastening section (112) which is configured for a force- and form-fitting connection with the culture vessel (5); and/or
- comprises a second fastening section (113) which is designed for a force- and form-fitting connection with the culture vessel (5).

6. Modular carrier unit (1) according to the preceding claim 5, wherein
- the first fastening section (112)
o is formed at the bottom (111b) and/or in the region of the bottom (111b) of the blind hole (111), and/or
o comprises projections and/or recesses for a positive connection; and/or
- the second fastening section (113)
o is formed at and/or in the region of the opening (111a), and/or
o comprises an elastic fastening element (114), in particular a rubber ring, for a force-fit connection, wherein the fastening element (114) is preferably arranged in a groove (116) formed in a side wall (111c) of the blind hole (111).

7. Modular carrier unit (1) according to any one of the preceding claims 1 to 6,
- wherein at least one of the side surfaces (S1, S2, S3, S4) comprises a second connection interface (102) which is designed for connecting the modular carrier unit (1) to a further modular carrier unit (1), and/or
- wherein the first connection interface (101) and/or the second connection interface (102) is designed for a positive-fit and/or force-fit connection.

8. Modular carrier unit (1) according to any one of the preceding claims 1 to 7, wherein
- the first connection interface (101) is designed differently from the second connection interface (102), or
- the first connection interface (101) corresponds to the second connection interface (102).

9. Modular carrier unit (1) according to any one of the preceding claims 1 to 8, wherein
- the first connection interface (101) is configured for connection to a second connection interface (102) of the further carrier unit (1) and/or
- the second connection interface (102) is configured for connection to a first connection interface (101) of the further carrier unit (1).

10. Modular carrier unit (1) according to any one of the preceding claims 1 to 9, wherein
- the first connection interface (101) for connection to the further carrier unit (1)
o has projections and/or recesses for a positive-fit connection and/or
o for a force-fit connection, comprises a magnet (115) and/or a spring system, and/or
- the second connection interface (102) for connection to the other carrier unit (1)
o has projections and/or recesses for a positive-fit connection and/or
o for a force-fit connection, comprises a magnet (115) and/or a spring system.

11. Modular carrier unit (1) according to any one of the preceding claims 1 to 10, comprising a removal handle (30) which is couplable to the carrier body (10) for the removal of a modular carrier unit connected to a further modular carrier unit (1), wherein the carrier body (10) preferably comprises a handle fastening section (31) which is designed for a form-fitting and/or force-fitting connection with the removal handle (30).

12. Modular carrier unit (1) according to any one of the preceding claims 1 to 11,
- wherein the carrier body (10) is cuboid and/or rectangular in shape, and/or
- wherein the drive transmission unit (20) is rotatably mounted relative to the carrier body (10) by means of a bearing unit (40),
- wherein the drive transmission unit (20) is designed as a gear transmission with at least one gear (21) or comprises a gear transmission with at least one gear (21),
- wherein the drive transmission unit (20) is designed as a belt and/or chain drive or comprises a belt and/or chain drive,
- wherein the drive transmission unit (20) is designed as an electromagnetic drive or comprises an electromagnetic drive.

13. Modular carrier unit (1) according to any one of the preceding claims 1 to 12, wherein the gear transmission comprises exactly one gear (21) arranged on a shaft (21a), wherein
- the shaft (21a) is rotatably mounted relative to the carrier body (10), and
- the shaft (21a) forms the first drive transmission interface (201) at one end, and
- the gear forms the second drive transmission interfaces (202).

14. A modular carrier unit (1) according to any one of the preceding claims 1 to 13, wherein the gear train
- comprises a central gear (21) arranged on a central gear shaft (21a), and the central gear shaft (21a) forms the first drive transmission interface (201) at one end, wherein the central gear shaft (21a) is rotatably mounted relative to the carrier body (10) and
- for each side surface (S1, S2, S3, S4) comprises at least one outer gear (22, 23, 24, 25) which meshes with the central gear (21), wherein the outer gears (22, 23, 24, 25) are each rotatably mounted relative to the carrier body (10) by means of an outer gear pin (22a, 23a, 24a, 25a).

15. A module unit (3) comprising at least two modular carrier units (1) according to any one of the preceding claims 1 to 14.

16. Module unit (3) according to the preceding claim 15, wherein at least one of the at least two modular carrier units (1) comprises a drive unit (6) according to claim 2 and at least one of the at least two modular carrier units (1) comprises a culture vessel receiving unit (110), in particular a culture vessel receiving unit (110) with a culture vessel (5), as per claim 3.

17. Incubator (2) comprising at least one modular carrier unit (1) according to any one of the preceding claims 1 to 14 and/or a module unit (3) according to any one of the preceding claims 15 or 16,
the incubator (2) preferably comprising a control unit (4) for controlling the modular carrier unit (1) and/or the module unit (3), wherein the control unit (4) is coupled to the modular carrier unit (1) and/or the module unit (3) via signal and/or control technology, in particular via a line (L) or wirelessly.

18. Use of the modular carrier unit (1) according to any one of the preceding claims 1 to 14 and/or the module unit (3) according to any one of the preceding claims 15 or 16 in an incubator (2).

## Revendications

1. Unité de support modulaire (1) pour un incubateur (2), en particulier pour une unité modulaire (3) d'un incubateur (2), comprenant :
- un corps de support (10) présentant
∘ une ouverture de réception (100), qui est réalisée pour la réception d'un récipient de culture (5) ou d'une unité d'entraînement (6), et
∘ trois, quatre ou plus de surfaces latérales (S1, S2, S3, S4),
∘ dans laquelle au moins une des surfaces latérales (S1, S2, S3, S4) présente une première interface de liaison (101), qui est réalisée pour la liaison de l'unité de support modulaire (1) à une autre unité de support modulaire (1),
- une unité de transmission d'entraînement (20), qui est réalisée pour la transmission d'un couple d'entraînement et est agencée au niveau du corps de support (10), l'unité de transmission d'entraînement (20) présentant :
∘ au moins une première interface de transmission d'entraînement (201), laquelle peut être couplée au récipient de culture (5) ou à l'unité d'entraînement (6),
o une deuxième interface de transmission d'entraînement (202), qui peut être couplée à une deuxième interface de transmission d'entraînement (202) d'une unité de transmission d'entraînement (20) de l'autre unité de support modulaire (1) ou de l'unité d'entraînement (6).

2. Unité de support modulaire (1) selon la revendication précédente 1, présentant l'unité d'entraînement (6), qui est fixée au corps de support (10) au niveau de et/ou dans l'ouverture de réception (100) et est couplée à la première interface de transmission d'entraînement (201) pour la transmission du mouvement de rotation de l'unité d'entraînement (6) à l'unité de transmission d'entraînement (20),
dans laquelle l'unité d'entraînement (6) est de préférence un moteur électrique, de préférence un moteur pas à pas.

3. Unité de support modulaire (1) selon la revendication précédente 1, présentant une unité de réception de récipient de culture (110) agencée de manière rotative dans l'ouverture de réception (100), laquelle unité de réception est réalisée pour la réception du récipient de culture (5) et est couplée à la première interface de transmission d'entraînement (201) pour la transmission du mouvement de rotation de l'unité de transmission d'entraînement (20) à l'unité de réception de récipient de culture (110),
dans laquelle de préférence un ou plusieurs récipients de culture (5) peuvent être agencés ou sont agencés dans l'unité de réception de récipient de culture (110).

4. Unité de support modulaire (1) selon la revendication précédente 3, dans laquelle l'unité de réception de récipient de culture (110) pour la réception du récipient de culture (5)
- est réalisée en forme de pot et/ou
- est réalisée en forme de cône et/ou
- présente un trou borgne (111), qui s'étend entre une ouverture (111a) et un fond (111b).

5. Unité de support modulaire (1) selon la revendication précédente 4, dans laquelle le trou borgne (111)
- présente une première section de fixation (112), qui est réalisée pour la liaison par force et par complémentarité de forme au récipient de culture (5) ; et/ou
- présente une deuxième section de fixation (113), qui est réalisée pour la liaison par force et par complémentarité de forme au récipient de culture (5).

6. Unité de support modulaire (1) selon la revendication précédente 5, dans laquelle
- la première section de fixation (112)
∘ est réalisée au niveau du fond (111b) et/ou dans la zone du fond (111b) du trou borgne (111), et/ou
o présente des saillies et/ou des évidements pour une liaison par complémentarité de forme ; et/ou
- la deuxième section de fixation (113)
∘ est réalisée au niveau de et/ou dans la zone de l'ouverture (111a), et/ou
o présente un élément de fixation (114) élastique, en particulier un anneau en caoutchouc, pour une liaison par force, dans laquelle l'élément de fixation (114) est agencé de préférence dans une rainure (116), qui est réalisée dans une paroi latérale (111c) du trou borgne (111).

7. Unité de support modulaire (1) selon l'une quelconque des revendications précédentes 1 à 6,
- dans laquelle au moins une des surfaces latérales (S1, S2, S3, S4) présente une deuxième interface de liaison (102), qui est réalisée pour la liaison de l'unité de support modulaire (1) à une autre unité de support modulaire (1), et/ou
- dans laquelle la première interface de liaison (101) et/ou la deuxième interface de liaison (102) est réalisée pour une liaison par complémentarité de forme et/ou par force.

8. Unité de support modulaire (1) selon l'une quelconque des revendications précédentes 1 à 7, dans laquelle
- la première interface de liaison (101) est réalisée de manière différente de la deuxième interface de liaison (102), ou
- la première interface de liaison (101) correspond à la deuxième interface de liaison (102).

9. Unité de support modulaire (1) selon l'une quelconque des revendications précédentes 1 à 8, dans laquelle
- la première interface de liaison (101) est réalisée pour la liaison à une deuxième interface de liaison (102) de l'autre unité de support (1) et/ou
- la deuxième interface de liaison (102) est réalisée pour la liaison à une première interface de liaison (101) de l'autre unité de support (1).

10. Unité de support modulaire (1) selon l'une quelconque des revendications précédentes 1 à 9, dans laquelle
- la première interface de liaison (101) pour la liaison à l'autre unité de support (1)
o présente des saillies et/ou des évidements pour une liaison par complémentarité de forme et/ou
o présente un aimant (115) et/ou un système ressort pour une liaison par force, et/ou
- la deuxième interface de liaison (102) pour la liaison à l'autre unité de support (1)
o présente des saillies et/ou des évidements pour une liaison par complémentarité de forme et/ou
o présente un aimant (115) et/ou un système ressort pour une liaison par force.

11. Unité de support modulaire (1) selon l'une quelconque des revendications précédentes 1 à 10, présentant une poignée de démontage (30), qui peut être couplée au corps de support (10) pour le démontage d'une unité de support modulaire (1) reliée à une autre unité de support modulaire (1), dans laquelle le corps de support (10) présente de préférence une section de fixation de poignée (31), qui est réalisée pour une liaison par complémentarité de forme et/ou par force à la poignée de démontage (30).

12. Unité de support modulaire (1) selon l'une quelconque des revendications précédentes 1 à 11,
- dans laquelle le corps de support (10) est réalisé en forme de cube et/ou en forme de parallélépipède, et/ou
- dans laquelle l'unité de transmission d'entraînement (20) est logée de manière rotative par rapport au corps de support (10) au moyen d'une unité de palier (40),
- dans laquelle l'unité de transmission d'entraînement (20) est réalisée sous forme de transmission par engrenage avec au moins une roue dentée (21) ou comprend une transmission par engrenage avec au moins une roue dentée (21),
- dans laquelle l'unité de transmission d'entraînement (20) est réalisée sous forme de transmission par courroie et/ou par chaîne ou comprend une transmission par courroie et/ou par chaîne,
- dans laquelle l'unité de transmission d'entraînement (20) est réalisée sous forme d'entraînement électromagnétique ou comprend un entraînement électromagnétique.

13. Unité de support modulaire (1) selon l'une quelconque des revendications précédentes 1 à 12, dans laquelle la transmission par engrenage présente exactement une roue dentée (21) agencée sur un arbre (21a), dans laquelle
- l'arbre (21a) est logé de manière rotative par rapport au corps de support (10), et
- l'arbre (21a) forme la première interface de transmission d'entraînement (201) au niveau d'une extrémité, et
- la roue dentée forme les deuxièmes interfaces de transmission d'entraînement (202).

14. Unité de support modulaire (1) selon l'une quelconque des revendications précédentes 1 à 13, dans laquelle la transmission par engrenage
- présente une roue dentée centrale (21) agencée sur un arbre de roue centrale (21a) et l'arbre de roue centrale (21a) forme la première interface de transmission d'entraînement (201) au niveau d'une extrémité, dans laquelle l'arbre de roue centrale (21a) est logé de manière rotative par rapport au corps de support (10) et
- présente au moins une roue dentée extérieure (22, 23, 24, 25) par surface latérale (S1, S2, S3, S4), qui vient en prise dans la roue dentée centrale (21), dans laquelle les roues dentées extérieures (22, 23, 24, 25) sont logées de manière rotative par rapport au corps de support (10) respectivement au moyen d'une broche de roue extérieure (22a, 23a, 24a, 25a).

15. Unité modulaire (3) présentant au moins deux unités de support modulaires (1) selon l'une quelconque des revendications précédentes 1 à 14.

16. Unité modulaire (3) selon la revendication précédente 15, dans laquelle au moins une des au moins deux unités de support modulaires (1) présente une unité d'entraînement (6) selon la revendication 2 et au moins une des au moins deux unités de support modulaires (1) présente une unité de réception de récipient de culture (110), en particulier une unité de réception de récipient de culture (110) avec un récipient de culture (5), selon la revendication 3.

17. Incubateur (2) présentant au moins une unité de support modulaire (1) selon l'une quelconque des revendications précédentes 1 à 14 et/ou une unité modulaire (3) selon l'une quelconque des revendications précédentes 15 ou 16,
l'incubateur (2) présentant de préférence une unité de commande (4) pour la commande de l'unité de support modulaire (1) et/ou de l'unité modulaire (3), dans lequel l'unité de commande (4) est couplée à l'unité de support modulaire (1) et/ou à l'unité modulaire (3) par la technique de signalisation et/ou par la technique de commande, en particulier par câble au moyen d'une ligne (L) ou sans fil.

18. Utilisation de l'unité de support modulaire (1) selon l'une quelconque des revendications précédentes 1 à 14 et/ou de l'unité modulaire (3) selon l'une quelconque des revendications précédentes 15 ou 16 dans un incubateur (2).
